# EUROPEAN PATENT SPECIFICATION

(11) **EP 1 786 808 B1**
(45) Date of publication and mention of the grant of the patent: **09.07.2008**
(21) Application number: 05773498.0
(22) Date of filing: 31.08.2005
(51) Int. Cl.: C07D 417/12

(54) **METHOD FOR THE PREPARATION OF ROSIGLITAZONE**
VERFAHREN ZUR HERSTELLUNG VON ROSIGLITAZON
METHODE POUR LA PREPARATION DE ROSIGLITAZONE

(30) Priority: 10.09.2004 CZ 20040956
(43) Date of publication of application: 23.05.2007
(73) Proprietor: Zentiva, a.s., 102 37 Praha 10 (CZ)
(72) Inventor: HALAMA, Ales, 530 09 Pardubice (CZ); LUSTIG, Petr, 530 03 Pardubice (CZ); JIRMAN, Josef, 100 00 Praha 10 (CZ)
(74) Representative: Jirotkova, Ivana
(86) International application number: PCT/CZ2005/000067
(87) International publication number: WO 2006/026934

(56) References cited:
- WO-A-93/13095
- WO-A-98/37073
- WO-A-03/029251
- WO-A-20/04007490
- WO-A-20/05021541
- WO-A-20/05073227
- SORBERA L A ET AL: "ROSIGLITAZONE MALEATE" DRUGS OF THE FUTURE, BARCELONA, ES, vol. 23, no. 9, 1998, pages 977-985, XP000856586 ISSN: 0377-8282

## Description

The invention relates to a new method of carrying out the reduction of 5-[4-[2-(N-methyl-N-, (2-pyridyl)-amino)ethoxy]benzylidene]thiazolidine-2,4-dione yielding rosiglitazone, i. e. a substance which is used for the preparation of a drug for the treatment of hyperglycaemia in patients who suffer from *diabetes mellitus* of second type.

### Background Art

Rosiglitazone, 5-[4-[2-(N-methyl-N-(2-pyridyl)amino)ethoxy]benzyl]thiazolidine-2,4-dione of formula I, is a well-known antihyperglycaemic, chemical synthesis of which was first described in patent EP306228 (1989) to Beecham. Rosiglitazone of formula I is usually prepared by the reduction of 5-[4-[2-(N-methyl-N-(2-pyridyl)amino)ethoxy]benzylidene]-thiazolidine-2,4-dione of formula II, see the following equation. The reduction of the compound of formula II to the substance of formula I may be carried out by various methods.

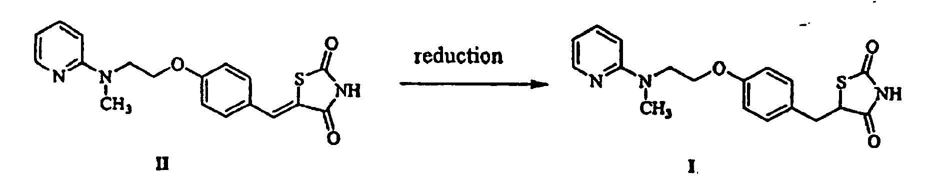

Usually, the reduction of the compound of formula II to the substance of formula I is carried out by catalytic hydrogenation in various media. Large consumption of very expensive catalysts, palladium on carbon in particular, is typical of those methods. Besides an excessive consumption of the catalyst, a high consumption of the solvent, caused by low solubility of the substance undergoing reduction, presents another disadvantage of this method. Furthermore, the reduction must be carried out under the pressure of hydrogen, for which special production equipment is needed. Carrying out of catalytic hydrogenations of the precursor of formula II is described in patent documents EP 306228 (1989) and WO 9923095.

Another method of the preparation of the substance of formula I was published in patent application WO 9837073. The reduction of the compound of formula II was carried out using complex lithium borohydrides under the catalysis of pyridine in tetrahydrofuran, e. g. LiB(secBu)₃H, LiBH₄, NaBH₄ in a mixture with LiCl.

Complex hydrides of metals (e. g. LiAlH₄ or NaBH₄) easily reduce polarized bonds like C=N, C=O etc. Only under very specific conditions they can reduce the C=C bond. In particular, borides and aluminides of transition metals (from the series of Ni, Co, Pd, and Rh) are used. The most of the data available in literature concerning the reduction of olefins are about an agent of NaBH₄/MX₂ type (MX₂ = CoCl₂), see Ganem B.: Chemical Reviews (1986), 86(5), 763-80.

In available literature, a possibility was described how to reduce olefins to alkanes using metal borohydrides under the catalysis of cobalt salts in the presence of complexing agents. Sodium borohydride was usually used as the reducing agent, but borohydrides of lithium, potassium, zinc and tetraalkylammonium could be used as well. Cobalt(II) chloride, cobalt(II) acetate and cobalt(III) chloride were used as the source of cobalt. Complexing agents from the series of dimethylglyoxime, 2,2'-bipyridyl a 1,10-phenanthroline serve as ligands. The reactions are usually carried out in solvents like alcohols, tetrahydrofuran, and dimethylformamide. These reductions are described in more detail in R. C. Larrock, "Comprehensive Organic Transformations", John Wiley & Sons, Inc, 1999, p. 7-8; Satoh T.: Chem. Pharm. Bull 1971, 19, 817; Chung S.K. J. Org. Chem. 1979, 44, 1014.

Reduction by sodium borohydride of the respective precursors of formula IV under the catalysis of cobalt salts in the presence of a suitable complexing agent yielded certain benzyl-2,4-thiazolidinediones (general formula III, Ar stands for a substituted aromate), which are substances related to rosiglitazone.

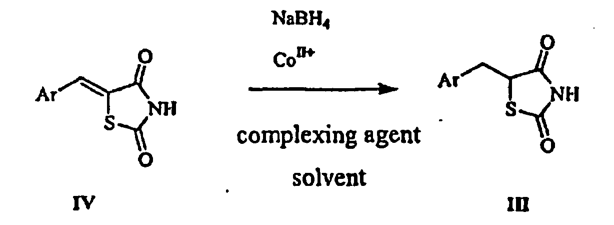

This arrangement of the reduction was published in patent documents WO 93/13095 and WO 04/007490 for the antihyperglycaemics pioglitazone, englitazone, and ciglitazone. Nevertheless, this method has not been described for the antihyperglycaemic rosiglitazone.

We submit a new and effective solution how to reduce the substance of formula II. The present method is economical and can be advantageously applied on an industrial scale. The method yields the antihyperglycaemic rosiglitazone of formula I in a quality needed for pharmaceutical substances.

### Disclosure of Invention

The invention relates to a new method of carrying out the reduction of 5-[4-[2-(N-methyl-N-(2-pyridyl)-amino)ethoxy]benzylidene]thiazolidine-2,4-dione of formula II, which consists in the use of metal borohydrides of general formula MBH₄, wherein M stands for a cation from the group of Na, Li, K, Zn and R₄N, and R stands for a C₁-C₅ alkyl group, under the catalysis of cobalt salts in the presence of at least one complexing agent and a suitable solvent, 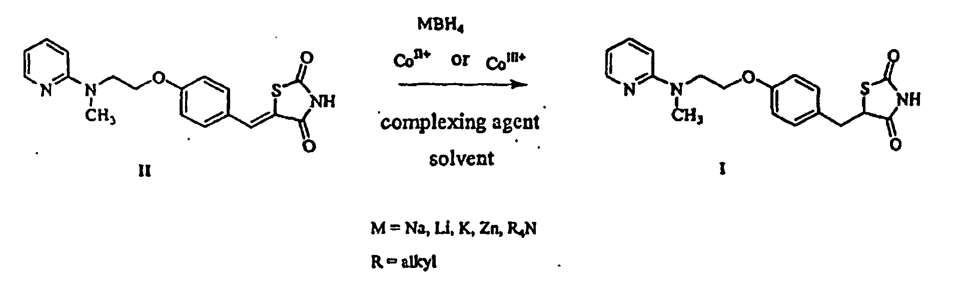 whereas the starting substance of formula II is dissolved in an aqueous solution of sodium hydroxide, and the following substances are added: a bivalent or trivalent cobalt salt, a complexing agent, dimethylformamide, and an aqueous solution of a reducing agent, particularly sodium borohydride; rosiglitazone of formula I is isolated from the reaction mixture by the procedure comprising the following steps:
a) Adding an organic solvent into the reaction mixture,
b) Adjusting pH of the reaction mixture to a value of 5 by adding a diluted acid, and
c) Adding a 10% aqueous solution of sodium hydrogen carbonate into the reaction mixture and filtering the precipitated product, and
d) Crystallization of crude rosiglitazone of formula I..

This method, as opposed to the previous solutions, does not require the use of expensive palladium-based catalysts and hydrogen as a reducing agent, which makes it possible to carry out the reaction without using pressure equipment. The substance of formula II in the form of the E-isomer or Z-isomer, or a mixture of both isomers (E/Z) in any ratio, or a mixture of tautomeric forms and solvates of the compound of formula II may be used as the starting material. The reduction is carried out in a suitable solvent at temperatures of the reaction medium of 20 to 75 °C. Water or water in a mixture with miscible organic solvents chosen from the series of dimethylformamide, C₁-C₅ alcohols, acetone and tetrahydrofuran serves as the reaction medium.

Sodium borohydride (M = Na) can be used advantageously as the reducing agent, but the reaction also proceeds when other borohydrides, e. g. of lithium, potassium, zinc and tetraalkylammonium, are used. Cobalt(II) chloride (CoCl₂), cobalt(II) acetate ((CH₃COO)₂Co), cobalt(II) nitrate (Co(NO₃)₂), and cobalt(III) chloride (CoCl₃) can be used as the source of cobalt. Cobalt(II) chloride is usually used either in the form of the hexahydrate (C_{O}Cl₂*6H₂O) or deposited on a silica gel as "blue silica gel". Complexing agents from the series of dimethylglyoxime, 2,2'-bipyridyl, and 1,10-phenanthroline serve as ligands.

The solvent used is preferably a CI-C5 alcohol, most preferably ethanol.

Crystallization of crude rosiglitazone of formula I is preferably carried out by dissolving the substance in a boiling solvent, filtering the hot solution, preferably through a sorbent layer, slowly cooling the filtrate under stirring, and filtering the crystalline product. The sorbent is most preferably chosen from the group of silicon(IV) oxide, aluminium oxide and activated carbon.

The process according to the invention preferably comprises the following steps:
[1] Preparation of the catalyst
[2] Preparation of a solution of the reducing agent
[3] Carrying out of the reduction itself
[4] Isolation of the crude product
[5] Crystallization

The preparation of the catalyst may include dissolving the cobalt salt and the complexing agent in a polar aprotic solvent from the group of dimethylformamide, dimethylacetamide, dimethyl sulfoxide, N-methylpyrrolidone, and hexamethylphosphoramide. The preparation of the solution of the reducing agent may include dissolving borohydrides of general formula MBH₄, where M and R are as defined in claim 1, in water or an aqueous solution of sodium hydroxide at a temperature of 0 to 20 °C. The reduction of the compound of formula II may include dissolving the compound of formula II in an aqueous solution of sodium hydroxide, and repeatedly adding the solution of the catalyst and, subsequently, that of the reducing agent at a temperature of 35 to 75 °C.

The following molar ratios of the reactants are advantageous: starting substance 1, cobalt salt 0.01 to 0.03, complexing agent 0.1 to 0.3, borohydride 1 to 3.

The reductions of the compound of formula II to the substance of formula I were, on the basis of the method we invented, carried out advantageously in the following way. First, the starting substance was dissolved in an aqueous solution of sodium hydroxide, and then a bivalent or trivalent cobalt salt, a complexing agent (most often dimethylglyoxime), and dimethylformamide were added. While the reaction mixture was being heated, an aqueous solution of the reducing agent, most often sodium borohydride, was being slowly added into it. It was found out that gradual adding of both the catalyst and the reducing agent in repeated doses favourably influences the conversion. The type of the cobalt salt used does not have a significant influence either on the conversion or on the yield of the product (see examples 1 to 4). Temperature plays an important role; the reaction proceeds very slowly at temperatures below 30 °C, an ideal temperature interval is 35 to 75 °C. The following molar ratios of the reactants were found out as ideal: starting substance 1, cobalt salt 0.01 to 0.03, complexing agent 0.1 to 0.3, and borohydride 1 to 3.

Advantageousness of our solution is especially clear when compared with WO 93/13095. That document describes experimental conditions for an analogous reduction for the series of benzylidene-2,4-thiazolinediones, particularly for the precursor of the antihyperglycaemic pioglitazone. The method used for pioglitazone in a mixed medium of water-tetrahydrofuran, when a crude product with a 95% yield was obtained (example 3 in WO 93/13095), is there described as the most suitable solution.

The described method usually leads with 70 to 90% yields to a free base of rosiglitazone of formula I, i. e. 5-[4-[2-(N-methyl-N-(2-pyridyl)amino)ethoxy]benzyl]thiazolidine-2,4-dione.

The method we have invented leads to the product of formula I, the chemical purity of which is 98 % and more, but usually more the 99.5 %. The method we have invented does not use, as opposed to hydrogenation reductions, expensive palladium-based catalysts, and does not require any pressure equipment for the production. When compared with those methods, our solution is more economical, and better industrially applicable.

The invention is clarified in greater detail in the following examples, which have no influence on the scope of the invention defined in the claims.

### EXAMPLES

### EXAMPLE 1

[1] Preparation of the catalyst: 0.2 g of cobalt(II) chloride hexahydrate and 1.33 g of dimethylglyoxime were dissolved in 20 ml of DMF yielding a clear blue-green solution (volume ca. 25 ml).
[2] Preparation of the solution of the reducing agent: 4.5 g of sodium borohydride was dissolved in 50 ml of a 0.1 M solution of sodium hydroxide under cooling in ice bath (the solution was, before its use, stored in a refrigerator, volume ca. 50 ml).
[3] Carrying out the reduction itself: 2.75 g of sodium hydroxide and, subsequently, 20 g of starting substance II were dissolved in 200 ml of water. The obtained solution was stirred and heated to a temperature of 55±5 °C. The catalyst was added dropwise (1/5 of the volume according to [1], during of ca. 2 minutes) into the solution, then the reducing agent was added (1/5 of the volume according to [2], during of ca. 5 minutes) and the mixture was subsequently stirred at 55±5 °C for 50 minutes. The adding of the catalyst (1/5 of the volume according to [I]) and the reducing agent (1/5 of the volume according to [2]), including the stirring and heating of the reaction mixture, was repeated four more times in the same way. The total reaction time was approximately 5 hours.
[4] Isolation of the crude product: 50 ml of ethyl acetate, 40 ml of hydrochloric acid (1:1), and, very slowly, 100 ml of a 10% solution of sodium hydrogen carbonate were added into the reaction mixture (according to [3]). The precipitated lumpy suspension was filtered, washed with water (2x100 ml) and ethanol (2x50 ml) and dried in a vacuum drier at 70 °C. The product with a melting point of 154-155 °C, and yield of 91 % was obtained. The content of cobalt in the product, determined using inductively coupled plasma mass spectrometry, is 8 ppm.
[5] Crystallization: The crude substance according to [4] was dissolved in boiling ethanol (450 ml), the hot solution was filtered through a sorbent layer (Al₂O₃ or SiO₂), and the filtered solution was, under stirring, allowed to cool freely. The precipitated crystals were sucked off, washed with ethanol (45 ml) and dried in a vacuum drier at 70 °C. The product with a melting point of 154.4-155.5 °C was obtained. The yield of crystallization was 86 %. The content of cobalt in the product, determined using inductively coupled plasma mass spectrometry, is 5 ppm.

### EXAMPLE 2

The reduction was carried out in the same way as in example 1 with the only difference that cobalt(II) chloride was replaced by the same amount of cobalt(II) acetate tetrahydrate. The crude product was obtained with a yield of 89 %. The yield of crystallization was 80 %.

### EXAMPLE 3

The reduction was carried out in the same way as in example 1 with the only difference that cobalt(II) chloride was replaced by the same amount of cobalt(II) nitrate hexahydrate. The crude product was obtained with a yield of 87 %. The yield of crystallization was 84 %.

### EXAMPLE 4

The reduction was carried out in the same way as in example 1 with the only difference that cobalt(II) chloride was replaced by the same amount of cobalt(III) chloride. The crude product was obtained with a yield of 85 %. The yield of crystallization was 82%.

### EXAMPLE 5

The reduction was carried out in the same way as in example 1 with the only difference that dimethylglyoxime was replaced by the same molar amount of 1,10-phenanthroline. The crude product was obtained with a yield of 86 %. The yield of crystallization was 85 %.

### EXAMPLE 6

The reduction was carried out in the same way as in example 1 with the only difference that sodium borohydride was replaced by the same molar amount of lithium borohydride (LiBH₄). The crude product was obtained with a yield of 90%. The yield of crystallization was 85 %.

### EXAMPLE 7

The reduction was carried out in the same way as in example 1 with the only difference that sodium borohydride was replaced by the same molar amount of tetraethylammonium borohydride (TEAB). The crude product was obtained with a yield of 87 %. The yield of crystallization was 82 %.

### EXAMPLE 8 (comparative example)

10g of substance II was dissolved in a solution of 1 g of sodium hydroxide and 150 ml of water. 2.2 g of cobalt(II) chloride deposited on silica gel, 0.4 g of dimethylglyoxime (DMG) and 14 ml of dimethylformamide (DMF) were added into the solution. The mixture was stirred and heated to 35 °C, and an aqueous solution of lithium borohydride (1.8 g) was gradually added into it. The temperature of the reaction mixture was kept in the interval of 35 to 45 °C for 3 hours. The catalyst was then filtered off, pH of the filtrate was adjusted to 6 using diluted hydrochloric acid (1:1), and ethyl acetate (80 ml) was added. Ethyl acetate was slowly evaporated, which led to the precipitation of a lumpy product. The product was filtered, dried, and crystallized from ethanol. White crystalline powder with a melting point of 154-155 °C and chemical purity of 99.7 % (HPLC) was obtained. The yield of rosiglitazone was 76 %. The content of cobalt in the product, determined using inductively coupled plasma mass spectrometry, is 220 ppm.

### EXAMPLE 9 (comparative example)

10g of substance II was dissolved in a solution of 1g of sodium hydroxide and 150 ml of water. A solution of 0.089 g of cobalt(II) chloride hexahydrate and 0.51 g of DGM dissolved in 10 ml DMF was added into the solution heated to 40 °C. The mixture was stirred and warmed to 35 - 45 °C and an aqueous solution of sodium borohydride (2.38g) was gradually added into it during eight hours. The pH of the reaction mixture was then adjusted to 6 using diluted hydrochloric acid (1:1), and the product was extracted with ethyl acetate. After evaporation of the solvent from the organic phase the residue was crystallized from ethanol. White crystalline powder with a melting point of 155-156 °C and chemical purity of 99.8 % (HPLC) was obtained. The yield of rosiglitazone was 70 %.

## Claims

1. A method for the preparation of rosiglitazone of formula I by reduction of 5-[4-[2-(N-methyl-N-(2-pyridyl)amino)ethoxy]-benzylidene]thiazolidine-2,4-dione of formula II, **characterized in that** the reduction is carried out using at least one borohydride of general formula MBH₄, wherein M stands for a cation from the group of Na, Li, K, Zn and R₄N, and R stands for a C₁-C₅ alkyl group, under the catalysis of cobalt salts in the presence of at least one complexing agent and a suitable solvent 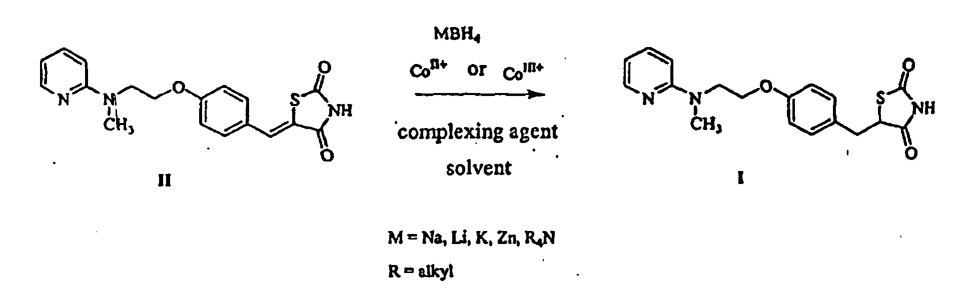 whereas the starting substance of formula II is dissolved in an aqueous solution of sodium hydroxide, and the following substances are added: a bivalent or trivalent cobalt salt, a complexing agent, dimethylformamide, and an aqueous solution of a reducing agent, particularly sodium borohydride; rosiglitazone of formula I is isolated from the reaction mixture by the procedure comprising the following steps:
a) Adding an organic solvent into the reaction mixture,
b) Adjusting pH of the reaction mixture to a value of 5 by adding a diluted acid, and
c) Adding a 10% aqueous solution of sodium hydrogen carbonate into the reaction mixture and filtering the precipitated product, and
d) Crystallization of crude rosiglitazone of formula I.

2. The method according to claim 1, **characterized in that** crystallization of the crude compound of formula I is carried out by dissolving the substance in a boiling solvent, filtering the hot solution, preferably through a sorbent layer, slowly cooling the filtrate under stirring, and filtering the crystalline product.

3. The method according to claim 1 or 2, **characterized in that** the source of cobalt is cobalt(II) chloride CoCl₂, cobalt(II) acetate (CH₃COO)₂Co, cobalt(II) nitrate, or cobalt(III) chloride CoCl₃, or hydrates of these salts.

4. The method according to claim 1; 2 or 3, **characterized in that** the complexing agent is chosen from the group of dimethylglyoxime, 2,2'-bipyridyl, and 1,10-phenanthroline.

5. The method according to any of claims 1 to 4, **characterized in that** the reaction is carried out at a temperature of the reaction mixture of 35 to 75 °C

6. The method according to any of claims 1 to 5, **characterized in that** it comprises the following steps:
[1] Preparation of the catalyst
[2] Preparation of a solution of the reducing agent
[3] Carrying out of the reduction itself
[4] Isolation of the crude product
[5] Crystallization

7. The method according to claim 6, **characterized in that** the preparation of the catalyst consists in dissolving the cobalt salt and the complexing agent in a polar aprotic solvent from the group of dimethylformamide, dimethylacetamide, dimethyl sulfoxide, N-methylpyrrolidone, and hexamethylphosphoramide.

8. The method according to claim 7, **characterized in that** the preparation of the solution of the reducing agent consists in dissolving borohydrides of general formula MBH₄, where M and R are as defined in claim 1, in water or an aqueous solution of sodium hydroxide at a temperature of 0 to 20 °C.

9. The method according to claim 8, **characterized in that** the reduction of the compound of formula II is carried out by dissolving the compound of formula II in an aqueous solution of sodium hydroxide, and repeatedly adding the solution of the catalyst and, subsequently, that of the reducing agent at a temperature of 35 to 75 °C.

10. The method according to any claim 1 to 9, **characterized in that** the solvent comprises a C₁-C₅ alcohol.

11. The method according to claim 10, **characterized in that** the solvent is ethanol.

12. The method according to claim 1 to 9, **characterized in that** the sorbent is chosen from the group of silicon(IV) oxide, aluminium oxide and activated carbon.

13. The method according to any of claims 1 to 12, **characterized in that** the following molar ratios of the reactants are used: starting substance 1, cobalt salt 0.01 to 0.03, complexing agent 0.1 to 0.3, borohydride 1 to 3.

## Patentansprüche

1. Verfahren zur Herstellung von Rosiglitazon der Formel I durch Reduktion von 5-[4-[2-(N-Methyl-N-(2-pyridyl)amino)ethoxy]-benzyliden]thiazolidin-2,4-dion der Formel II, **dadurch gekennzeichnet, dass** die Reduzierung unter Gebrauch zumindest eines Borohydrids der allgemeinen Formel MBH₄ durchgeführt wird, wobei M das Kation aus der Reihe Na, Li, K, Zn und R₄N und R ein C₁-C₅ Alkyl darstellen, unter der Katalyse von Salzen zweiwertiger oder dreiwertiger Kobalt in der Anwesenheit von zumindest einem Komplexbildner und geeigneten Lösungsmittel, 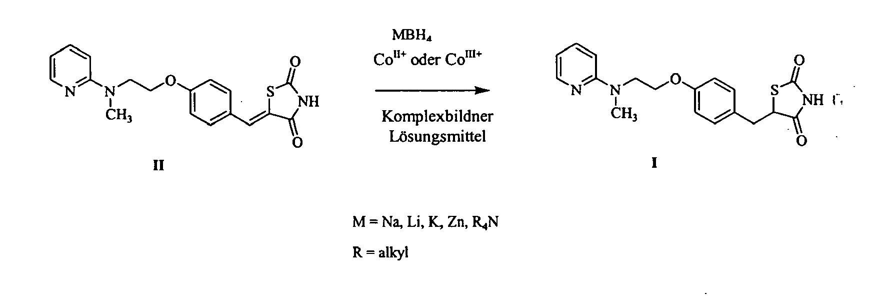 wobei der Ausgangsstoff der Formel II in einer wässerigen Lösung des Natriumhydroxids aufgelöst wird und die folgenden Stoffe hinzugegeben werden: zwei- oder dreiwertiges Kobaltsalz, der Komplexbildner, Dimethylformamid und eine wässerige Lösung des Reduktionsmittels, insbesondere Natriumborohydrid; Rosiglitazon der Formel I wird aus der Reaktionsmischung durch einen die nachfolgenden Schritte enthaltenden Vorgang isoliert:
a) Zugabe eines organischen Lösungsmittels zur Reaktionsmischung
b) Nachstellung des pH der Reaktionsmischung auf den Wert 5 durch Zugabe einer verdünnten Säure und
c) Zugabe von einer 10% wässerigen Lösung von Natrium-Hydrogenkarbonat zur Reaktionsmischung und Filtration des ausgeschiedenen Produktes und
d) Kristallisierung des rohen Rosiglitazons der Formel I.

2. Verfahren nach Anspruch 1, **dadurch gekennzeichnet, dass** die Kristallisierung der rohen Verbindung der Formel I durch Auflösung des Stoffes im kochenden Lösungsmittel, Filtration der heißen Lösung, vorzugsweise durch eine Sorbensschicht, langsame Abkühlung des Filtrates unter Rühren und Filtration des kristallinen Produktes durchgeführt wird.

3. Verfahren nach Anspruch 1 oder 2, **dadurch gekennzeichnet, dass** die Kobaltquelle Kobaltdichlorid CoCl₂, Kobaltacetat (CH₃COO)₂Co, Kobaltnitrat oder Kobalttrichlorid CoCl₃, oder Hydrate dieser Salze, sind.

4. Verfahren nach Anspruch 1, 2 oder 3, **dadurch gekennzeichnet, dass** der Komplexbildner aus der Reihe von Dimethylglyoxim, 2,2'-Bipyridyl und 1,10-Phenanthrolin gewählt wird.

5. Verfahren nach einem der Ansprüche 1 bis 4, **dadurch gekennzeichnet, dass** die Reaktion bei Temperaturen der Reaktionsmischung von 35 bis 75 °C durchgeführt wird.

6. Verfahren nach einem der Ansprüche 1 bis 5, **gekennzeichnet durch** die folgenden Schritte:
[1] Vorbereitung des Katalysators
[2] Vorbereitung der Reduktionsmittellösung
[3] Durchführung der eigentlichen Reduktion
[4] Isolierung des Rohproduktes
[5] Kristallisierung

7. Verfahren nach Anspruch 6, **dadurch gekennzeichnet, dass** die Vorbereitung des Katalysators aus Auflösung des Kobaltsalzes und Komplexbildners in einem polaren, aprotischen Lösungsmittel aus der Reihe von Dimethylformamid, Dimethylacetamid, Dimethylsulfoxid, N-Methylpyrrolidon und Hexamethylphosphoramid besteht.

8. Verfahren nach Anspruch 7, **dadurch gekennzeichnet, dass** die Vorbereitung der Reduktionsmittellösung aus Auflösung des Borohydrids allgemeiner Formel MBH₄, wobei M und R die in Anspruch 1 definierte Bedeutung haben, in Wasser oder in einer wässerigen Lösung des Natriumhydroxid unter einer Temperatur von 0 bis 20 °C besteht.

9. Verfahren nach Anspruch 8, **dadurch gekennzeichnet, dass** die Reduktion der Verbindung der Formel II durch Auflösen der Verbindung der Formel II in einer wässerigen Lösung des Natriumhydroxid und wiederholten Zusatz der Katalysatorslösung und nachfolgend der Reduktionsmittellösung bei Temperaturen in einem Umfang von 35 bis 75 °C durchgeführt wird.

10. Verfahren nach einem der Ansprüche 1 bis 9, **dadurch gekennzeichnet, dass** das Lösungsmittel einen C₁-C₅ Alkohol umfasst.

11. Verfahren nach Anspruch 10, **dadurch gekennzeichnet, dass** das Lösungsmittel Äthanol ist.

12. Verfahren nach Ansprüchen 1 bis 9, **dadurch gekennzeichnet, dass** das Sorbens aus der Reihe von Siliziumdioxid, Aluminiumoxid und Aktivkohle gewählt ist.

13. Verfahren nach einem der Ansprüche 1 bis 12, **dadurch gekennzeichnet, dass** man die folgenden molaren Verhältnisse der Reaktanten verwendet: Ausgangsstoff 1, Kobaltsalz 0,01 bis 0,03, Komplexbildner 0,1 bis 0,3, Borohydrid 1 bis 3.

## Revendications

1. Procédé de préparation de la rosiglitazone de formule I par réduction de la 5-[4-[2-(N-méthyl-N-(2-pyridyl)amino)éthoxy]-benzylidène]thiazolidine-2,4-dione de formule II, **caractérisé en ce que** l'on effectue la réduction en utilisant au moins un borohydrure de formule générale MBH₄, où M indique un cation du groupe Na, Li, K, Zn et R₄N et R indique un groupe alkyle C₁-C₅, avec catalyse par des sels de cobalt en présence d'au moins un agent complexant et d'un solvant approprié, 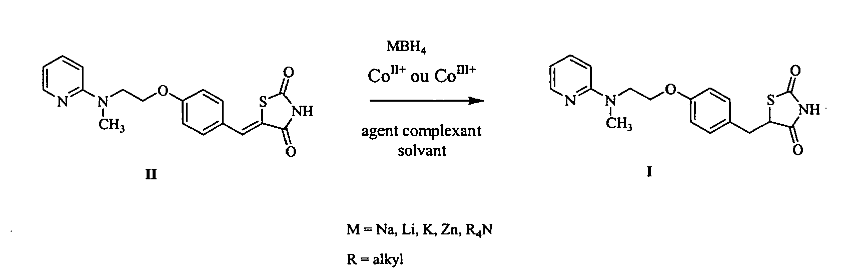 la substance de départ de formule II étant dissolue dans une solution aqueuse d'hydroxyde de sodium et les substances suivantes étant ajoutées: le sel de cobalt bivalent ou trivalent, l'agent complexant, la diméthylformamide et une solution aqueuse d'un réactif réducteur, en particulier du borohydrure de sodium; la rosiglitazone de formule I étant isolée du mélange réactionel par un procédé comportant les étapes suivantes :
a) ajouter du solvant organique au mélange réactionel;
b) ajuster le pH du mélange réactionel à la valeur 5 en ajoutant un acide dilué; et
c) ajouter une solution aqueuse à 10% de bicarbonate de sodium au mélange réactionel et filtrer le précipité ; et
d) cristallisation de la rosiglitazone brute de formule I.

2. Procédé selon la revendication 1, caractérissé en ce que l'on fait la cristallisation du composé brut de formule 1 par dissolution de la substance en solvant bouillant, filtration de la solution chaude, de préférence à travers une couche d'absorbant, refroidissement lent du filtrat avec agitation et filtration du produit cristallin.

3. Procédé selon la revendication 1 ou 2, **caractérisé en ce que** la source de cobalt est le chlorure de cobalt bivalent CoCl₂, l'acétate de cobalt bivalent (CH₃COO)₂Co, le nitrate de cobalt bivalent ou le chlorure de cobalt trivalent CoCl₃, ou des hydrates de ces sels.

4. Procédé selon la revendication 1, 2 ou 3, **caractérisé en ce que** l'agent complexant est choisi parmi le grooupe de diméthylglyoxime, 2,2'-bipyridyle et 1,10-phénanthroline.

5. Procédé selon l'une quelconque des revendications 1 à 4, **caractérisé en ce que** l'on effectue la réaction à une température du milieu de réaction entre 35 et 75 °C.

6. Procédé selon l'une quelconque des revendications 1 à 5, **caractérisé en ce qu'**il comporte les étapes suivantes :
[1] Préparation du catalyseur ;
[2] Préparation de la solution d'agent réducteur ;
[3] Réalisation de la réduction ;
[4] Isolation du produit brut ;
[5] Cristallisation.

7. Procédé selon la revendication 6, **caractérisé en ce que** la préparation du catalyseur consiste en dissolution du sel de cobalt et de l'agent complexant dans un solvant polaire aprotique choisi parmi diméthylformamide, diméthylacétamide, diméthylsulfoxide, N-méthylpyrrolidone et hexaméthylphosphoramide.

8. Procédé selon la revendication 7, **caractérisé en ce que** la préparation de la solution d'agent réducteur consiste en dissolution du borohydrure de formule générale MBH₄, où M et R sont tels que définis dans la revendication 1, dans de l'eau ou dans une solution aqueuse d'hydroxyde de sodium sous une température entre 0 et 20°C.

9. Procédé selon la revendication 8, **caractérisé en ce que** l'on effectue la réduction du composé de formule II en dissolvant le composé de formule II dans une solution aqueuse d'hydroxyde de sodium et en y ajoutant en plusieurs fois la solution de catalyseur puis celle de l'agent réducteur sous une température entre 35 et 75 °C.

10. Procédé selon l'une quelconque des revendications 1 à 9, **caractérisé en ce que** le solvant comprend un alcool C₁-C₅.

11. Procédé selon la revendication 10, **caractérisé en ce que** le solvant est de l'éthanol.

12. Procédé selon les revendications 1 à 9, **caractérisé en ce que** l'absorbant est choisi parmi l'oxyde de silicium, l'oxyde d'aluminium et le charbon actif.

13. Procédé selon l'une quelconque des revendications 1 à 12, **caractérisé en ce que** l'on utilise des quantités molaires des réactifs suivantes: substance de départ 1; sel de cobalt 0,01 à 0,03, l'agent complexant 0,1 à 0,3, borohydrure 1 à 3.
